# EUROPEAN PATENT APPLICATION

(11) **EP 4 524 175 A1**
(43) Date of publication of application: **19.03.2025**
(21) Application number: 23803758.4
(22) Date of filing: 03.05.2023
(51) Int. Cl.: C08G 63/06, C08G 63/78, C08G 63/80, C08J 11/24, C07C 69/82, C07C 69/84, D01F 6/62

(54) **METHOD FOR PRODUCING POLYESTER RESIN AND FIBER COMPRISING REGENERATED BIS(2-HYDROXYETHYL) TEREPHTHALATE**

(30) Priority: 09.05.2022 KR 20220056794
(71) Applicant: SK Chemicals Co., Ltd., Seongnam-si, Gyeonggi-do 13494 (KR)
(72) Inventor: PARK, Jun-Yong, Seongnam-si, Gyeonggi-do 13494 (KR); KIM, Do-Kyoon, Seongnam-si, Gyeonggi-do 13494 (KR); KIM, Sung-Gi, Seongnam-si, Gyeonggi-do 13494 (KR); KIM, Ji-Hun, Seongnam-si, Gyeonggi-do 13494 (KR); PARK, Kwang-Woo, Seongnam-si, Gyeonggi-do 13494 (KR); PARK, Ji Hye, Seongnam-si, Gyeonggi-do 13494 (KR); LEE, Joong Ki, Seongnam-si, Gyeonggi-do 13494 (KR)
(74) Representative: Dehns
(86) International application number: PCT/KR2023/006075
(87) International publication number: WO 2023/219335

(57) **Abstract**

A polyester resin according to the present invention comprises a regenerated bis(2-hydroxyethyl) terephthalate having high purity and containing diethylene glycol ester in an amount controlled below a certain level, and thus has heat resistance and quality comparable to the original resin even though the polyester resin is regenerated through chemical recycling. In particular, a polyester fiber comprising the polyester resin of the present invention has excellent strength, elongation, and processability, and can thus be used as an eco-friendly fiber product.

## Description

### Technical Field

The present invention relates to processes for preparing a polyester resin and a polyester fiber comprising recycled bis(2-hydroxyethyl) terephthalate.

### Background Art

Polyester is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world are preparing regulations and plans for recycling waste plastic resources, including waste polyester. For example, there is an attempt to use a recycled resin in packaging materials used in various fields at a certain ratio or more. Although physical or chemical methods are used as methods of recycling waste polyester, physical recycling methods cannot guarantee purity and are not widely used.

In chemical recycling methods, the ester bond of waste polyester is cleaved to depolymerize it. Reactions such as glycolysis, hydrolysis, methanolysis, and aminolysis are used. Glycolysis among them is to decompose waste polyester by adding a glycol such as ethylene glycol or diethylene glycol at high temperatures. A reaction product containing mainly bis(2-hydroxyethyl) terephthalate (BHET) is obtained. The bis(2-hydroxyethyl) terephthalate may be used as a raw material for preparing unsaturated polyester or ester polyol after the crystallization or purification thereof.

### [Prior Art Document]

(Patent Document 1) Korean Patent No. 1386683
(Patent Document 2) U.S. Patent No. 7211193

### Disclosure of Invention

### Technical Problem

In general, a product of the depolymerization reaction of a waste polyester resin comprises a significant amount of oligomers such as dimers and trimers in addition to bis(2-hydroxyethyl) terephthalate (BHET). Since diethylene glycol (DEG) is formed at a high depolymerization temperature, it is inevitable that side-reaction products derived from diethylene glycol are formed. For example, a diethylene glycol ester (DEG ester) causes a decrease in physical properties in polymerizing a regenerated polyester resin, the extent of which is greater in the case of polyester copolymers. In addition, it makes the color of the polymerization raw materials dark and yellow; thus, the use thereof is inevitably limited.

As a result of research conducted by the present inventors to solve this problem, it was possible to prepare recycled BHET with high purity in which the content of diethylene glycol esters is controlled to a certain level or lower by carrying out the depolymerization reaction in multiple stages, while significantly lowering the temperature of the latter stage, thereby reducing the formation of diethylene glycol and impurities derived therefrom and to prepare polyester resins and fibers with excellent thermal resistance and quality using the same.

Accordingly, an object of the present invention is to provide a polyester resin and a polyester fiber comprising recycled bis(2-hydroxyethyl) terephthalate with high purity and high quality and processes for preparing the same.

### Solution to Problem

According to the present invention, there is provided a process for preparing a polyester resin, which comprises (1) depolymerizing waste polyester to prepare recycled bis(2-hydroxyethyl) terephthalate, wherein the recycled bis(2-hydroxyethyl) terephthalate has a peak area fraction of bis(2-hydroxyethyl) terephthalate of 96% or more and a peak area fraction of a diethylene glycol ester of less than 2% in total when measured by high-performance liquid chromatography (HPLC); (2) mixing the recycled bis(2-hydroxyethyl) terephthalate with a solvent to prepare a solution of recycled bis(2-hydroxyethyl) terephthalate; (3) subjecting the recycled bis(2-hydroxyethyl) terephthalate solution to a first polycondensation reaction under a pressure of 200 mmHg to 600 mmHg to prepare an oligomer; and (4) subjecting the oligomer to a second polycondensation reaction under a pressure of less than 200 mmHg to prepare a polyester resin.

In addition, according to the present invention, there is provided a polyester resin prepared by the above process and having an intrinsic viscosity of 0.6 dl/g to 1.2 dl/g.

In addition, according to the present invention, there is provided a polyester fiber, which comprises the polyester resin.

In addition, according to the present invention, there is provided a process for preparing a polyester fiber, which comprises spinning the polyester resin to obtain a fiber.

### Advantageous Effects of Invention

Since the polyester resin according to the present invention has high purity and comprises recycled bis(2-hydroxyethyl) terephthalate in which the content of diethylene glycol esters is adjusted to a certain level or less, even though it is a polyester resin regenerated through chemical recycling, it has thermal resistance and quality comparable to a virgin resin.

In particular, the polyester fiber comprising the polyester resin of the present invention is excellent in strength, elongation, and processability; thus, it can be used as an environmentally friendly fiber product.

### Best Mode for Carrying out the Invention

Hereinafter, the present invention will be described in more detail.

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element and/or component, unless specifically stated to the contrary.

The process for preparing a polyester resin of the present invention comprises (1) depolymerizing waste polyester to prepare recycled bis(2-hydroxyethyl) terephthalate, wherein the recycled bis(2-hydroxyethyl) terephthalate has a peak area fraction of bis(2-hydroxyethyl) terephthalate of 96% or more and a peak area fraction of a diethylene glycol ester of less than 2% in total when measured by high-performance liquid chromatography (HPLC); (2) mixing the recycled bis(2-hydroxyethyl) terephthalate with a solvent to prepare a solution of recycled bis(2-hydroxyethyl) terephthalate; (3) subjecting the recycled bis(2-hydroxyethyl) terephthalate solution to a first polycondensation reaction under a pressure of 200 mmHg to 600 mmHg to prepare an oligomer; and (4) subjecting the oligomer to a second polycondensation reaction under a pressure of less than 200 mmHg to prepare a polyester resin.

Bis(2-hydroxyethyl) terephthalate is an ester of two ethylene glycols and one terephthalic acid. For example, it is a compound formed as an intermediate in the process of preparing polyester such as polyethylene terephthalate (PET) through the polymerization of ethylene glycol and terephthalic acid or its ester.

In particular, bis(2-hydroxyethyl) terephthalate (BHET), which is used as a polymerization raw material for the polyester resin according to the present invention, is obtained from waste polyester having a repeat unit of ethylene glycol and terephthalic acid like polyethylene terephthalate (PET) or glycol-modified polyethylene terephthalate (PETG). For example, it may be obtained by well-known depolymerization methods such as glycolysis, hydrolysis, and methanolysis.

In the present specification, bis(2-hydroxyethyl) terephthalate (BHET) obtained by the depolymerization of waste polyester as described above is referred to as "recycled bis(2-hydroxyethyl) terephthalate (recycled BHET)," or abbreviated as r-BHET or rBHET, which needs to be understood as distinct from a pure BHET compound.

Specifically, recycled BHET may contain reagents or solvents used in various chemical steps during the depolymerization of waste polyester, or by-products formed by side reactions with them. These impurities may remain in trace amounts even after several rounds of purification. Thus, recycled BHET generally contains trace amounts of organic and inorganic impurities in addition to BHET as the main component. For this reason, recycled BHET can also be viewed as a kind of composition comprising two or more components, i.e., a BHET composition. It may be used as a polymerization raw material for producing a polyester resin.

According to an embodiment, the recycled bis(2-hydroxyethyl) terephthalate in step (1) may be prepared by a process, which comprises (1a) subjecting waste polyester to depolymerization by a first glycolysis reaction at a temperature of 180°C to 200°C to obtain a first reactant; (1b) subjecting the first reactant to depolymerization by a second glycolysis reaction at a temperature of 150°C to 170°C to obtain a second reactant; (1c) subjecting the second reactant to ion exchange through an ion-exchange resin to obtain a third reactant; (1d) removing an unreacted glycol from the third reactant through distillation at a temperature of 150°C or lower to obtain a fourth reactant; and (1e) subjecting the fourth reactant to distillation to obtain crude bis(2-hydroxyethyl) terephthalate.

Hereinafter, each step will be described in detail.

### Preparation of waste polyester

Waste polyester used as a raw material in the present invention may be obtained from a polyester material product discarded after use.

For example, the waste polyester may be obtained from products such as beverage bottles, fabrics, films, cases, boxes, partitions, shelves, protective panels, packaging materials, building materials, and interior and exterior materials made of various polyester materials discarded after having been used by consumers.

The waste polyester material may be pretreated before being subjected to a depolymerization step.

First, once other plastics, metals, and foreign substances mixed in the waste polyester material have been removed, it is washed and screened according to detailed characteristics such as color, if necessary.

The waste polyester material thus screened is put into a pulverizer to be pulverized into small flakes. The flakes of waste polyester thus obtained may be screened to a desired particle size or less using a mesh. The size of the mesh may be, for example, 4 mm or less, 3 mm or less, or 2 mm or less.

The screened flakes may be washed, dried by hot air or the like, and then put into a depolymerization step.

Meanwhile, the waste polyester subjected to a glycolysis reaction may have a controlled particle size. For example, the waste polyester may be pulverized by the pretreatment step as described above to have a flake shape.

Specifically, the particle diameter of the waste polyester may be 4 mm or less, 3 mm or less, 2 mm or less, or 1 mm or less. Within the above particle diameter range, a glycolysis reaction under relatively low-temperature conditions is possible. For example, the temperature condition of the first glycolysis reaction may be adjusted to 195°C or lower, 190°C or lower, 185°C or lower, or 180°C, and the temperature condition of the subsequent second glycolysis reaction may be adjusted to 160°C or lower or 150°C or lower. In addition, within the above particle diameter range, a glycolysis reaction within a relatively short period of time is possible. For example, the period of time for the first and second glycolysis reactions may be 3 hours or less, 2 hours or less, or 1 hour or less, from the point when the appropriate temperature is reached.

In addition, the waste polyester may have a fine structure like a fiber. For example, the waste polyester may be a waste fiber or a fibrous material such as a waste banner.

As a specific example, the waste polyester may have a particulate or fibrous form with a particle diameter of 4 mm or less.

The fiber may comprise at least one of a monofilament yarn and a multifilament yarn. The diameter of the monofilament yarn may be, for example, 0.05 denier to 100 denier and may correspond to approximately 0.001 mm to 0.1 mm. Specifically, the monofilament yarn may have a diameter of 0.05 denier to 7 denier or 7 denier to 100 denier. The diameter of the multifilament yarn may be, for example, 1 denier to 10,000 denier and may correspond to approximately 0.01 mm to 1 mm. Specifically, the multifilament yarn may have a diameter of 0.01 denier to 0.2 denier or 0.2 denier to 1 denier.

If the depolymerization is carried out as the particle diameter or diameter of waste polyester is adjusted within the specific range, solvation can be expedited even under the conditions of relatively low temperatures and short reaction time.

In particular, according to the subject invention, a two-stage glycolysis reaction (i.e., a first glycolysis reaction and a second glycolysis reaction) is carried out. If the solvation is expedited in the first glycolysis reaction, the transesterification reaction (ester exchange reaction) of the waste polyester can be performed under the conditions of lower temperatures and short reaction time in the second glycolysis reaction. Thus, it is possible to significantly reduce the concentration of diethylene glycol (DEG) naturally formed at a common glycolysis reaction temperature and to significantly reduce the content of diethylene glycol ester compounds (DEG esters) in the bis(2-hydroxyethyl) terephthalate finally prepared.

The diethylene glycol ester compounds present in bis(2-hydroxyethyl) terephthalate acts as a factor that breaks the regularity of a final polymer during the subsequent polymerization of a polyester and a polyester copolymer, thereby deteriorating the thermal resistant characteristics of the final polymer such as melting point (Tm), glass transition temperature (Tg), and the like.

However, polyester resins and products manufactured using bis(2-hydroxyethyl) terephthalate obtained according to the depolymerization process of the present invention can be polymerized into polymers, without unnecessary structural defects, as in the case where a virgin, non-recycled raw material is used.

### Depolymerization

According to the process of the present invention, as waste polyester is subjected to the pretreatment of pulverization and to a depolymerization reaction in multiple stages at low temperatures, it is possible to significantly reduce the content of glycol dimers (diethylene glycol) formed during the depolymerization reaction, so that there is an advantage in that the purity of bis(2-hydroxyethyl) terephthalate finally obtained is increased and side-reaction structures in a subsequent repolymerization into polyester are minimized.

According to an embodiment, the depolymerization comprises subjecting waste polyester to depolymerization through a first glycolysis reaction at a high temperature (180 to 200°C) to obtain a first reactant; and subjecting the first reactant to depolymerization at a low temperature (150 to 170°C) through a second glycolysis reaction to obtain a second reactant.

As is well known, the glycolysis reaction refers to a chemical reaction in which a polymer chain or the like is decomposed by a glycol. The glycol may comprise, for example, at least one selected from the group consisting of ethylene glycol, propylene glycol, and diethylene glycol.

A catalyst may be used in the glycolysis reaction. The catalyst may be a metal catalyst, for example, a metal salt catalyst or a metallic organic catalyst. Specifically, the catalyst may be an acetate, carbonate, oxide, or hydroxide of a metal, and the metal may be an alkali metal, an alkaline earth metal, or a transition metal.

As a specific example, the catalyst comprises a metal acetate or an anhydride or a hydride thereof. More specifically, it may be at least one selected from the group consisting of zinc acetate, sodium acetate, cobalt acetate, and manganese acetate, or in the form of a hydrate or anhydride thereof.

The total weight of the glycol added may be 1, 2, or 3 times the weight of the waste polyester resin or more and may be 7, 5, or 4 times or less. For example, the weight of the glycol added may be 1 to 7 times, specifically, 2 to 5 times, more specifically, 3 to 4 times, relative to the weight of the waste polyester resin.

In addition, the weight of the catalyst added may be 0.01 part by weight or more, 0.1 part by weight or more, 0.2 part by weight or more, or 0.3 part by weight or more, and may be 5 parts by weight or less, 1 part by weight or less, 0.7 part by weight or less, 0.5 part by weight or less, or 0.4 part by weight or less, relative to 100 parts by weight of the waste polyester resin. For example, the weight of the catalyst added may be 0.1 part by weight to 1 part by weight, specifically, 0.2 part by weight to 0.7 part by weight, relative to 100 parts by weight of the waste polyester resin. More specifically, the catalyst may be used in an amount of 0.2 part by weight to 0.4 part by weight relative to 100 parts by weight of the waste polyester.

The temperature during the first glycolysis reaction may be 170°C or higher, 180°C or higher, or 190°C or higher, and may be 205°C or lower, 200°C or lower, 195°C or lower, or 190°C or lower. For example, the temperature during the first glycolysis reaction may be 180°C to 200°C, specifically, 180°C to 195°C, more specifically, 180°C to 190°C.

In addition, the temperature during the second glycolysis reaction may be 140°C or higher, 150°C or higher, or 160°C or higher, and may be 170°C or lower or 160°C or lower. For example, the temperature during the second glycolysis reaction may be 150°C to 170°C, specifically, 150°C to 160°C, more specifically, 150°C to 155°C.

The period of time required for the first and second glycolysis reactions may be 1 hour or more or 2 hours or more, and may be 4 hours or less or 3 hours or less, from the point when the appropriate temperature is reached. For example, the period of time required for the first and second glycolysis reactions may be 1 hour to 4 hours, specifically, 1 hour to 3 hours, more specifically, 1 hour to 2 hours, from the point when the appropriate temperature is reached.

As a specific example, the first glycolysis reaction may be carried out at a temperature of 180°C to 190°C for 1 hour to 3 hours. In addition, the second glycolysis reaction may be carried out at a temperature of 150°C to 160°C for 1 hour to 3 hours.

As an example, the first glycolysis reaction may be carried out in the presence of a zinc acetate anhydride catalyst. As a specific example, the first glycolysis reaction may be carried out at a temperature of 180°C to 200°C for 1 hour to 3 hours in the presence of a zinc acetate anhydride catalyst. The zinc acetate anhydride may be used in an amount of 0.2 part by weight to 0.4 part by weight relative to 100 parts by weight of the waste polyester. In addition, the second glycolysis reaction may be carried out at a temperature of 140°C to 160°C for 1 hour to 3 hours upon the further addition of ethylene glycol without an additional catalyst.

### Cooling and filtration

The second reactant obtained through the depolymerization may then be cooled and used in the next step.

The cooling temperature may be, for example, 150°C or lower, 140°C or lower, 130°C or lower, 120°C or lower, 110°C or lower, 100°C or lower, and may be 50°C or higher, 60°C or higher, 70°C or higher, 80°C or higher, or 90°C or higher.

As an example, the cooling may be carried out through a reduced pressure flash process. Specifically, the temperature of the second reactant may be lowered by evaporating ethylene glycol by applying a vacuum through the reduced pressure flash process.

For example, the second reactant may be further subjected to a step of cooling to 120°C or lower through reduced pressure flash prior to subsequent steps. More specifically, the temperature of the second reactant may be lowered to 110°C or lower or 100°C or lower by the reduced pressure flash process.

The pressure condition of the reduced pressure flash process may be, for example, 200 Torr or less, 100 Torr or less, or 50 Torr or less, specifically, 10 Torr to 200 Torr, 10 Torr to 100 Torr, or 10 Torr to 50 Torr.

Thereafter, insoluble foreign substances may be removed from the cooled second reactant through filtration. As a specific example, prior to the ion exchange in step (3), a step of cooling the second reactant to 120°C or lower and filtering it upon the addition of a filter aid may be further carried out. As a result, fine particles and insoluble organic substances present in the second reactant can be filtered out by solid-liquid separation.

Known ingredients such as diatomaceous earth, perlite, and asbestos powder may be used as the filter aid. For example, 0.1 part by weight to 2.0 parts by weight of the filter aid may be added to 100 parts by weight of the second reactant.

Since bis(2-hydroxyethyl) terephthalate (BHET) or oligomers obtained through the depolymerization reaction is present in a solid form at room temperature, it is difficult to separate foreign substances at room temperature. Thus, it is preferable to separate them at a temperature condition of 90°C to 150°C, more specifically, 110°C to 150°C. In addition, if the above temperature range is maintained, the removal of insoluble foreign substances may be facilitated thanks to good flowability.

Various methods and devices may be used in the removal of insoluble foreign substances through solid-liquid separation. For example, a device such as a pressurized filter, a centrifugal separator, a filter press, a belt press, or the like may be used. But it is not limited thereto as long as any method capable of separating foreign substances is used.

### Ion exchange

The second reactant, which has been depolymerized, cooled, and filtered, is subjected to ion exchange through an ion-exchange resin to obtain a third reactant.

As it is subjected to the ion exchange, ionic impurities present in the second reactant, specifically, catalysts and foreign substances, may be removed.

As is well known, an ion-exchange resin refers to a resin or polymer that serves as a medium for ion exchange. The ion-exchange resin may comprise a cation-exchange resin, an anion-exchange resin, an amphoteric ion-exchange resin, a chelate resin, or the like.

The cation-exchange resin may comprise a strongly acidic cation-exchange resin having a sulfonic acid group (-SO₃H) and a weakly acidic cation-exchange resin having a carboxyl group (-COOH). The anion-exchange resin may comprise a strongly basic anion-exchange resin in the form of a quaternary ammonium salt and a weakly basic anion-exchange resin having a primary to tertiary amino group.

As a specific example, the ion-exchange resin may comprise at least one selected from the group consisting of a strongly acidic cation-exchange resin, a weakly acidic cation-exchange resin, and a chelate resin.

According to an embodiment, the ion exchange is carried out by adding an ion-exchange resin to the second reactant.

The weight of the ion-exchange resin added may be 1, 3, or 5 times the weight of the catalyst added in the depolymerization reaction or more, and may be 20, 15, 10, or 8 times or less. For example, the weight of the ion-exchange resin added may be 1 to 20 times, specifically, 3 to 15 times, more specifically, 5 to 8 times, relative to the weight of the catalyst added in the depolymerization reaction.

In addition, the weight of the ion-exchange resin added may be 1 part by weight or more, 3 parts by weight or more, or 5 parts by weight or more, and may be 50 parts by weight or less, 20 parts by weight or less, 15 parts by weight or less, 10 parts by weight or less, or 7 parts by weight or less, relative to 100 parts by weight of the waste polyester resin employed in the depolymerization reaction.

As a specific example, the ion-exchange resin may be used in an amount of 1 part by weight to 20 parts by weight relative to 100 parts by weight of the waste polyester.

According to another embodiment, the ion exchange is carried out by using a column containing an ion-exchange resin.

Specifically, the column may be filled with particles of an ion-exchange resin, and ion exchange may be carried out while the second reactant passes through the column.

The particle diameter of the ion-exchange resin particles may be, for example, 0.3 mm to 1.5 mm, more specifically, 0.6 mm to 0.9 mm.

The temperature for ion exchange may be, for example, 140°C or lower, 130°C or lower, 120°C or lower, 110°C or lower, 100°C or lower, and may be 50°C or higher, 60°C or higher, 70°C or higher, 80°C or higher, or 90°C or higher.

### Removal and recovery of unreacted glycol

An unreacted glycol is removed from the third reactant through distillation to obtain a fourth reactant.

Since an unreacted glycol still remains in the depolymerization resultant after filtration thereof in the previous step, it is necessary to remove the same from the reactant prior to the next step.

In addition, it is necessary to perform a step of recovering an unreacted glycol for an economical depolymerization process. That is, it is possible to recover and reuse a glycol, among glycols such as ethylene glycol, propylene glycol, diethylene glycol, or the like previously employed in the depolymerization, that remains without participating in the glycolysis reaction.

The distillation to remove the unreacted glycol may be carried out by, for example, vacuum distillation. A glass distillation apparatus or a rotary evaporator may be used for this purpose.

As the vacuum distillation to remove the unreacted glycol is carried out at a temperature of 150°C or lower, the purity of BHET can be enhanced by further reducing the formation of diethylene glycol and impurities derived therefrom. For example, the vacuum distillation to remove the unreacted glycol may be carried out at a temperature of 150°C lower, 130°C lower, or 120°C lower, and 80°C higher, 90°C higher, 100°C higher, or 110°C higher. Specifically, the temperature during the distillation to remove the unreacted glycol may be 80°C to 190°C or 90°C to 150°C. As a more specific example, the distillation to remove the unreacted glycol may be carried out at a temperature of 100°C to 130°C.

The pressure during the distillation to remove the unreacted glycol may be, for example, 0.1 Torr to 200 Torr, more specifically, 0.5 Torr to 30 Torr.

### Distillation

The fourth reactant from which the unreacted glycol has been removed is subjected to distillation to obtain crude bis(2-hydroxyethyl) terephthalate.

Various methods may be used for the distillation, while a distillation method to make a mixture to be separated into a thin film for increasing its surface area in contact with a heat source may be used.

For example, the distillation may be carried out by thin film evaporation, falling film evaporation, or short path evaporation. For this purpose, a thin film evaporator, a falling film evaporator, and a short path evaporator may be used, respectively.

As a specific example, the distillation to obtain bis(2-hydroxyethyl) terephthalate may be carried out by thin film evaporation. Specifically, a mixture fed to the evaporator of the thin film evaporator forms a thin film on the inner wall of the thin film evaporator by the wiper rotor. Then, distillation is carried out under appropriate temperature conditions by heating. In addition, a condenser for recovering the evaporated material may be provided inside the thin film evaporator.

The thin film evaporation may be carried out by short path evaporation. Since such a short path and thin film evaporation has a short residence time and enables vacuum distillation using a high vacuum, it is possible to separate high-boiling or high-molecular-weight materials that are hardly separated by other distillation methods while minimizing the change of the reactants by heat. In addition, if the pressure inside a thin film evaporator is lowered, there is an advantage in that the vapor pressure of a material is reduced, which allows evaporation to take place at a lower temperature than its original boiling point.

As a specific example, the fourth reactant is fed to a short path and thin film evaporator, and a wiper for forming a thin film is rotated at 300 rpm or more. As a result, a vaporized material and a non-vaporized material can be separated from each other.

The internal thin film temperature of the upper thin film evaporation apparatus during the thin film evaporation may be, for example, 100°C or higher, 110°C or higher, 120°C or higher, or 125°C or higher, and may be 250°C or lower, 200°C or lower, 150°C or lower, or 135°C or lower, specifically, 150°C to 250°C, 190°C to 250°C, or 180°C to 220°C.

In addition, the internal pressure of the upper thin film evaporation apparatus during the thin film evaporation may be, for example, 0.005 Torr to 5.0 Torr, specifically, 0.05 Torr to 5.0 Torr, 0.05 Torr to 1.5 Torr, or 0.05 Torr to 1 Torr. More specifically, the distillation to obtain crude bis(2-hydroxyethyl) terephthalate may be carried out by thin film evaporation under a pressure of 0.05 Torr to 0.4 Torr.

### Adsorption-crystallization

The crude bis(2-hydroxyethyl) terephthalate is subjected to an adsorption-crystallization step to be provided as bis(2-hydroxyethyl) terephthalate with high purity and high quality.

For example, the adsorption-crystallization may be carried out by adding an adsorbent using water as a solvent, followed by filtration and crystallization.

Various solvents may be used for the adsorption-crystallization, but a solvent capable of dissolving bis(2-hydroxyethyl) terephthalate is preferably used as a solvent. As a specific example, in order to obtain the final reactant, water as a solvent is added to the crude bis(2-hydroxyethyl) terephthalate, which is dissolved by heating, and an adsorbent is added thereto, followed by subjecting the solution obtained by filtration to cooling-crystallization and final filtration. As a result, bis(2-hydroxyethyl) terephthalate with high purity can be obtained.

Water may be added in an amount of 100 parts by weight to 500 parts by weight, specifically, 200 parts by weight to 400 parts by weight, more specifically, 300 parts by weight to 350 parts by weight, relative to 100 parts by weight of the crude bis(2-hydroxyethyl) terephthalate.

In addition, the dissolution temperature may be 50°C to 95°C, specifically, 60°C to 85°C, more specifically, 70°C to 75°C.

The adsorbent added may serve to adsorb and remove other foreign substances. It may be added in an amount of 0.1 part by weight to 3 parts by weight relative to 100 parts by weight of the crude bis(2-hydroxyethyl) terephthalate. The type and form of the adsorbent are not particularly limited. For example, activated carbon may be used.

### Recycled bis(2-hydroxyethyl) terephthalate

Bis(2-hydroxyethyl) terephthalate, that is, recycled BHET, finally obtained through the above steps has high purity, along with organic impurities, especially diethylene glycol and by-products derived therefrom (DEG esters or the like) or other oligomers and inorganic substances, at a certain level or less, and it has excellent color quality.

This can be confirmed by analyzing the final product obtained from the above process with high-performance liquid chromatography (HPLC) and calculating the relative ratio between peak areas for the respective components. Specifically, the content of each component can be derived by measuring the fraction (%) of a peak area out of the total peak area in a spectrum obtained using high-performance liquid chromatography.

For example, the recycled bis(2-hydroxyethyl) terephthalate may have a peak area fraction (that is, purity) of bis(2-hydroxyethyl) terephthalate of 90% or more, 93% or more, or 95% or more when measured by HPLC.

According to an embodiment, the recycled bis(2-hydroxyethyl) terephthalate may have a peak area fraction of bis(2-hydroxyethyl) terephthalate of 96% or more when measured by HPLC. Specifically, the peak area fraction of bis(2-hydroxyethyl) terephthalate may be 96.5% or more, 97% or more, 97.5% or more, or 98% or more.

In addition, the recycled bis(2-hydroxyethyl) terephthalate may have a peak area fraction of organic impurities measured by HPLC of less than 5% in total, specifically, less than 4%, less than 3%, less than 2%, less than 1%, or less than 0.7%.

According to an embodiment, the recycled bis(2-hydroxyethyl) terephthalate may have a peak area fraction of diethylene glycol ester compounds of less than 2% in total when measured by HPLC. Specifically, the peak area fraction of diethylene glycol ester compounds may be less than 1.5%, less than 1.2%, less than 1%, less than 0.9%, less than 0.8%, or less than 0.7%, in total. More specifically, it may be 0% to less than 1.5%, 0% to less than 1%, or 0% to less than 0.8%.

The diethylene glycol ester compounds may be, for example, a condensate between an aromatic dicarboxylic acid such as terephthalic acid and diethylene glycol. As another example, the diethylene glycol ester compounds may be a condensate between an aromatic dicarboxylic acid such as terephthalic acid and a glycol (e.g., ethylene glycol) in addition to diethylene glycol. As a specific example, the diethylene glycol ester compounds may comprise at least one of 2-hydroxyethyl[2-(2-hydroxyethoxy)ethyl] terephthalate and bis[2-(2-hydroxyethoxy)ethyl]benzene-1,4-dicarboxylate.

In addition, the recycled bis(2-hydroxyethyl) terephthalate may have a peak area fraction of oligomers of 3% or less in total when measured by HPLC. This is a remarkably superior level in terms of purity and quality as compared with the oligomeric materials present in a product obtained by depolymerization in the conventional methods, which ranges from 10 to 20%.

Specifically, the recycled bis(2-hydroxyethyl) terephthalate may have a peak area fraction of BHET dimers of less than 3%, less than 2%, less than 1%, or less than 0.7%, when measured by HPLC. In addition, the recycled bis(2-hydroxyethyl) terephthalate may have a peak area fraction of BHET trimers of less than 1%, less than 0.5%, less than 0.3%, less than 0.1%, or 0%, when measured by HPLC.

In addition, the final product may further comprise impurities having a structure similar to that of bis(2-hydroxyethyl) terephthalate. For example, it may comprise at least one selected from the group consisting of monohydroxyethyl terephthalic acid (MHET), bis(2-hydroxypropyl) terephthalate, and monohydroxyethylethoxy terephthalic acid. The impurities having a structure similar to that of bis(2-hydroxyethyl) terephthalate may have a peak area fraction of less than 3%, less than 2%, less than 1%, or less than 0.5%, when measured by HPLC.

In addition, the recycled bis(2-hydroxyethyl) terephthalate may have a yellow index (YID) of 3.0 or less as measured with a spectrophotometer. Specifically, the yellow index may be 2.5 or less, 2.0 or less, 1.5 or less, or 1.0 or less.

In addition, the recycled bis(2-hydroxyethyl) terephthalate may have a total content of inorganic substances of less than 5 ppm as measured by inductively coupled plasma atomic emission spectroscopy (ICP-AES). Specifically, the total content of inorganic substances may be less than 3 ppm, less than 1 ppm, or nearly 0 ppm.

### Preparation of a solution of recycled bis(2-hydroxyethyl) terephthalate

The recycled bis(2-hydroxyethyl) terephthalate prepared above is mixed with a solvent to prepare a solution of recycled bis(2-hydroxyethyl) terephthalate.

Examples of the solvent used for preparing the solution of recycled BHET include water, ethylene glycol, methanol, and ethanol. As a specific example, the solvent may comprise at least one of water and ethylene glycol. As a more specific example, the solvent may comprise water and ethylene glycol at the same time, in which case the mixing weight ratio of water and ethylene glycol may be 1:5 or 1:2.

The temperature (dissolution temperature) at which the recycled BHET is mixed with the solvent may be, for example, 60°C or higher, 65°C or higher, 70°C or higher, or 75°C or higher, and may be 197°C or lower, 180°C or lower, 165°C or lower, 140°C or lower, 120°C or lower, 100°C or lower, 90°C or lower, or 80°C or lower.

According to an embodiment, the temperature at which the recycled BHET is mixed with water may be 60°C to 100°C. According to another embodiment, the temperature at which the recycled BHET is mixed with ethylene glycol may be 60°C to 197°C. According to an embodiment, the temperature at which the recycled BHET is mixed with a mixed solvent of water and ethylene glycol may be 60°C to 197°C. Within the preferred temperature range, it is more advantageous for preventing the problem of deterioration in purity due to the formation of diethylene glycol esters caused by thermal decomposition at a high temperature while the recycled BHET is sufficiently dissolved.

For example, the concentration of the solution of recycled BHET may be 25% by weight or more, 35% by weight or more, 50% by weight or more, 65% by weight or more, or 75% by weight or more, and may be 99% by weight or less, 95% by weight or less, 90% by weight or less, 85% by weight or less, or 80% by weight or less. As a specific example, the concentration of the solution of recycled BHET may be 25% by weight to 99% by weight, more specifically, 50% by weight to 95% by weight. Within the above preferred range, it may be more advantageous for inducing a uniform polymerization reaction with excellent reaction efficiency. The concentration of the solution of recycled BHET may be calculated as a percentage of the weight of the recycled BHET based on the total weight of the solution (i.e., the total weight of the recycled BHET and the solvent).

### Polymerization of a polyester resin

The solution of recycled bis(2-hydroxyethyl) terephthalate prepared above is used to polymerize a polyester resin.

The polymerization of the polyester resin in the present invention comprises carrying out a polycondensation reaction (first polycondensation reaction) under a low vacuum to prepare an oligomer of a low molecular weight; and subjecting the oligomer to a polycondensation reaction (second polycondensation reaction) under a high vacuum to prepare a polyester resin.

The first polycondensation reaction and the second polycondensation reaction may be carried out under a reduced pressure condition to discharge the solvent contained in the solution of recycled bis(2-hydroxyethyl) terephthalate and by-products (glycols or the like) of the polycondensation reaction to the outside of the system.

The pressure during the first polycondensation reaction may be, for example, 700 mmHg or less, 600 mmHg or less, 500 mmHg or less, 400 mmHg or less, 350 mmHg or less, 300 mmHg or less, or 250 mmHg or less, and may be 160 mmHg or more, 180 mmHg or more, 200 mmHg or more, 220 mmHg or more, or 240 mmHg or more. According to an embodiment, the pressure during the first polycondensation reaction is 200 mmHg to 600 mmHg. Within the above preferred range, it may be more advantageous for sufficiently removing by-products of the polycondensation reaction under a low vacuum while maintaining the degree of vacuum during the polycondensation reaction.

In addition, the temperature during the first polycondensation reaction may be, for example, 100°C or higher, 130°C or higher, 160°C or higher, 180°C or higher, or 200°C or higher, and may be 300°C or lower, 280°C or lower, 250°C or lower, or 230°C or lower. As a specific example, the first polycondensation reaction may be carried out at a temperature of 180°C to 250°C and a pressure of 200 mmHg to 400 mmHg.

In addition, the first polycondensation reaction may be carried out until the number average molecular weight of the oligomer of a low molecular weight reaches an appropriate level. The period of time required for the first polycondensation reaction is not particularly limited, but it may be, for example, 30 minutes or more, 1 hour or more, 2 hours or more, or 3 hours or more, and may be 15 hours or less, 10 hours or less, 5 hours or less, or 4 hours or less. Specifically, it may be 1 hour to 5 hours.

The pressure during the second polycondensation reaction may be, for example, less than 200 mmHg, 150 mmHg or less, 100 mmHg or less, 50 mmHg or less, 10 mmHg or less, or 1 mmHg or less, and may be 0.001 mmHg or more, 0.01 mmHg or more, 0.1 mmHg or more, or 0.5 mmHg or more. According to an embodiment, the pressure during the second polycondensation reaction is less than 200 mmHg. Within the above preferred range, it may be more advantageous for sufficiently removing by-products of the polycondensation reaction while maintaining the degree of vacuum during the polycondensation reaction.

In addition, the temperature during the second polycondensation reaction may be, for example, 230°C or higher, 240°C or higher, 250°C or higher, or 260°C or higher, and may be 300°C or lower, 290°C or lower, 280°C or lower, or 270°C or lower. As a specific example, the second polycondensation reaction may be carried out at a temperature of 250°C to 300°C and a pressure of 0.01 mmHg to 150 mmHg. Within the above preferred range, it may be more advantageous for sufficiently removing by-products of the polycondensation reaction, thereby suppressing the yellowing of a final resin, while maintaining the degree of vacuum during the polycondensation reaction.

In addition, the second polycondensation reaction may be carried out until the number average molecular weight of the polyester resin reaches an appropriate level. The period of time required for the second polycondensation reaction is not particularly limited, but it may be, for example, 30 minutes or more, 1 hour or more, 2 hours or more, or 5 hours or more, and may be 60 hours or less, 48 hours or less, 24 hours or less, or 15 hours or less. Specifically, it may be 1 hour to 24 hours.

The process for preparing a polyester resin of the present invention may further comprise a step commonly employed in this field in addition to the steps described above.

As an example, the process for preparing a polyester resin may further comprise molding the polyester resin to form pellets after step (4).

As another example, the process for preparing a polyester resin may further comprise subjecting the polyester resin to a solid-state polymerization after step (4). The temperature during the solid-state polymerization may be, for example, 180°C or higher, 190°C or higher, 200°C or higher, or 205°C or higher, and may be 260°C or lower, 240°C or lower, 220°C or lower, or 215°C or lower. As a specific example, the solid-state polymerization may be carried out at a temperature of 200°C to 220°C. In addition, the pressure during the solid-state polymerization may be, for example, 10.0 Torr or less, 5.0 Torr or less, 2.0 Torr or less, or 1.0 Torr or less, and may be 0.01 Torr or more, 0.1 Torr or more, 0.2 Torr or more, or 0.5 Torr or more. Specifically, it may be 0.2 Torr to 2.0 Torr. In addition, the solid-state polymerization may be carried out in an inert gas atmosphere such as nitrogen.

The polyester resin according to the present invention may be prepared as a copolymerized polyester resin by further adding an additional diacid component in addition to the recycled bis(2-hydroxyethyl) terephthalate. The additional diacid component may be a dicarboxylic acid or a derivative thereof. The dicarboxylic acid may comprise at least one selected from terephthalic acid and isophthalic acid. For example, a dicarboxylic acid or a derivative thereof may be additionally added during the first polycondensation reaction.

In addition, the polycondensation reaction may be carried out in the presence of a polycondensation catalyst. The polycondensation catalyst may be selected for use from a group consisting of a titanium-based compound, a germanium-based compound, an antimony-based compound, and an aluminum-based compound. The amount of the polycondensation catalyst used is preferably 0.1 ppm to 500 ppm based on the amount of metal elements relative to the weight of a final polyester resin. Since the amount used has an impact on the color of the final polyester resin, the amount used may vary depending on the desired color and the stabilizer and colorant used.

In addition to the polycondensation catalyst, a stabilizer, a colorant, a crystallizing agent, an antioxidant, a branching agent, or the like may be further used. The timing of adding these additives is not particularly limited, and they may be added at any time during the preparation step of the polyester resin.

As the stabilizer, phosphorus-based compounds such as phosphoric acid, trimethyl phosphate, triethyl phosphate, and triethyl phosphonoacetate may be generally used. The amount thereof added may be such that 10 to 200 ppm relative to the weight of the polyester resin based on the amount of elements. In addition, common colorants such as cobalt acetate and cobalt propionate may be exemplified as the colorant added to enhance the color of the polyester resin. The amount thereof added may be such that 10 to 200 ppm relative to the weight of the polyester resin based on the amount of cobalt element. If necessary, anthraquinone-based compounds, perinone-based compounds, azo-based compounds, methine-based compounds, or the like may be used as an organic colorant. Commercially available toners such as Polysynthren Blue RLS from Clarient or Solvaperm Red BB from Clarient may be used. The amount of the organic compound colorant added may be adjusted to 0 to 50 ppm based on the weight of the polyester resin. A crystal nucleating agent, an ultraviolet absorber, a polyolefin resin, a polyamide resin, and the like may be exemplified as the crystallizing agent. Hindered phenol-based antioxidants, phosphite-based antioxidants, thioether-based antioxidants, or mixtures thereof may be exemplified as the antioxidant. Conventional branching agents having three or more functional groups, for example, trimellitic anhydride, trimethylol propane, trimellitic acid, or mixtures thereof may be exemplified as the branching agent.

### Composition and characteristics of the polyester resin

The polyester resin of the present invention is a polyester resin regenerated through the chemical recycling of waste polyester.

Specifically, since the polyester resin of the present invention is polymerized using recycled BHET, it comprises a repeat unit derived from recycled BHET in the polymer chain.

The content of recycled BHET in the polyester resin of the present invention may be 90% by weight or more or 95% by weight or more. In addition, the content of recycled BHET may be 100% by weight or less, 99% by weight or less, or 95% by weight or less.

As an example, the recycled bis(2-hydroxyethyl) terephthalate may be employed in an amount of 90% by weight to 100% by weight based on the weight of the polyester resin.

Meanwhile, since bis(2-hydroxyethyl) terephthalate has a structure in which two ethylene glycols and one terephthalic acid are bonded, the polyester resin of the present invention may essentially comprise a repeat unit derived from ethylene glycol and terephthalic acid.

In addition, the polyester resin of the present invention may be a copolymerized polyester resin. For example, it may further comprise an additional diacid component as a copolymerization monomer. The additional diacid component may be a dicarboxylic acid or a derivative thereof. The dicarboxylic acid may comprise at least one selected from terephthalic acid and isophthalic acid. They may enhance the physical properties such as thermal resistance, chemical resistance, and weather resistance of a polyester resin.

As described above, as the polyester resin of the present invention comprises recycled bis(2-hydroxyethyl) terephthalate of high purity and high quality, it has low impurities and excellent thermal resistance even though it is a regenerated resin.

Accordingly, the present invention provides a polyester resin prepared by the process described above. That is, in the polyester resin of the present invention, the peak area fraction of bis(2-hydroxyethyl) terephthalate is 96% or more, and the peak area fraction of diethylene glycol (DEG) ester compounds is less than 2% in total, when measured by high-performance liquid chromatography (HPLC).

The intrinsic viscosity of the polyester resin of the present invention at 35°C may be, for example, 0.5 dl/g or more, 0.6 dl/g or more, or 0.7 dl/g or more, and may be 1.2 dl/g or less, 1.1 dl/g or less, 1.0 dl/g or less, or 0.9 dl/g or less. According to an embodiment, the polyester resin may have an intrinsic viscosity at 35°C of 0.6 dl/g to 1.2 dl/g. The intrinsic viscosity may be calculated, for example, by dissolving the polyester resin in a solvent such as orthochlorophenol and obtaining specific viscosity using an Ubbelohde viscometer or the like.

In addition, the polyester resin of the present invention may have a melting point (Tm) of, for example, 240°C or higher, 245°C or higher, 250°C or higher, or 255°C or higher, and may be 170°C or lower, 265°C or lower, or 260°C or lower. According to an embodiment, the polyester resin may have a melting point of 255°C or higher. The melting point may be measured by, for example, a method comprising putting the polyester resin in a differential scanning calorimeter (DSC) and raising the temperature from room temperature to 280°C at a constant rate.

In addition, the polyester resin may have, for example, a concentration of diethylene glycol of 2.5% by weight or less, 1.5% by weight or less, 1.2% by weight or less, 1.0% by weight or less, or 0.9% by weight or less, when measured by gas chromatography. As a specific example, the polyester resin may have a concentration of diethylene glycol of 0.8% by weight or less when measured by gas chromatography.

### Polyester fiber and process for preparing the same

Since the polyester resin has excellent strength, elongation, and processability when spun into a fiber as described above, it can be used in the preparation of a polyester fiber.

Accordingly, the present invention provides a polyester fiber, which comprises the polyester resin prepared by the process described above. That is, the polyester fiber of the present invention is prepared from a polyester resin, which comprises recycled bis(2-hydroxyethyl) terephthalate in which the peak area fraction of bis(2-hydroxyethyl) terephthalate is 96% or more, and the peak area fraction of diethylene glycol (DEG) ester compounds is less than 2% in total, when measured by high-performance liquid chromatography (HPLC).

The process for preparing a polyester fiber according to an embodiment of the present invention comprises (1) depolymerizing waste polyester to prepare recycled bis(2-hydroxyethyl) terephthalate, wherein the recycled bis(2-hydroxyethyl) terephthalate has a peak area fraction of bis(2-hydroxyethyl) terephthalate of 96% or more and a peak area fraction of a diethylene glycol ester of less than 2% in total when measured by high-performance liquid chromatography (HPLC); (2) mixing the recycled bis(2-hydroxyethyl) terephthalate with a solvent to prepare a solution of recycled bis(2-hydroxyethyl) terephthalate; (3) subjecting the recycled bis(2-hydroxyethyl) terephthalate solution to a first polycondensation under a pressure of 200 mmHg to 600 mmHg to prepare an oligomer; (4) subjecting the oligomer to a second polycondensation under a pressure of less than 200 mmHg to prepare a polyester resin; and (5) spinning the polyester resin to obtain a fiber.

Steps (1) to (4) may be carried out under the same processes and conditions as described in the process for preparing a polyester resin.

The spinning of the polyester resin in step (5) may comprise (5a) drying the polyester resin; (5b) melt-spinning the dried polyester resin to obtain an undrawn yarn; and (5c) drawing the undrawn yarn. It may further comprise (5d) thermally treating the drawn yarn. Alternatively, it may comprise melt-spinning the dried polyester resin to obtain a thermally treated drawn yarn; that is, it may comprise obtaining a drawn polyester yarn by simultaneously carrying out steps (5b), (5c), and (5d).

Specifically, the spinning step may be carried out by melt-spinning the polyester resin composition. It may be carried out using a melt spinning apparatus conventionally used, such as a spinning machine of a single- or twin-screw extruder type.

The temperature of the spinning step is preferably as low as possible while being a temperature sufficient to melt the polyester resin from the viewpoint of suppressing gelation. For example, the spinning step may be carried out under the condition that the temperature of the polyester resin discharged from the spinneret is 275°C to 300°C and may be carried out in a nitrogen atmosphere.

A spinneret commonly used for melt spinning may be used as the spinneret. For example, a spinneret having a discharge hole diameter of 0.1 mmϕ to 1.0 mmϕ and a discharge hole depth of about 0.1 mm to 5.0 mm may be used. The cross-sectional shape of a fiber for spinning is not particularly limited, but it may be a conventional one such as circular cross-section, triangular cross-section, rectangular cross-section, Y-shaped cross-section, cross-shaped cross-section, C-shaped cross-section, hollow cross-section, or grid-shaped cross-section.

The fiber discharged from the spinneret is generally cooled by exposure to wind at a wind speed of 5 m/minute to 100 m/minute after discharge and then wound. In this procedure, an oiling agent may be added as a bundling agent. The winding speed may be 300 m/minute to 5,000 m/minute.

This process may be carried out by the steps of spinning the polyester resin composition at a low speed to obtain an undrawn yarn (UDY) or spinning the polyester resin composition at a high speed to obtain a partially oriented yarn (POY); winding the undrawn or partially oriented yarn; and drawing the wound undrawn yarn or partially oriented yarn to obtain a fully drawn yarn (FDY). Alternatively, it may be carried out by the step of supplying the wound undrawn yarn, partially oriented yarn, or fully drawn yarn into a twisting machine; and twisting and drawing it to obtain a drawn-textured yarn.

Alternatively, the spinning step may be carried out by the step of directly spinning and drawing the polyester resin composition to obtain a spin drawn yarn (SDY) in one step.

In addition, it may be carried out by the steps of spinning the polyester resin composition to obtain an undrawn fiber, drawing the undrawn fiber, applying a serrated crimp to the drawn fiber, and cutting the crimped continuous fiber to obtain a staple fiber.

The staple fiber may have an average single yarn diameter of 0.5 denier to 5 denier. A crimped continuous fiber having a serrated crimp in the longitudinal direction of the fiber having a crimp (or repetitive bending) frequency of 50 crimps or less per centimeter may be obtained.

In addition, the polyester resin composition may be melt-spun through a spinneret to form a filament, which is cooled and solidified through a water-cooled cooling apparatus and an air-cooled cooling apparatus installed directly under the spinneret, stretched through a hot water bath at a temperature higher than Tg, and then heat-set to obtain a monofilament in one step in one machine.

The monofilament fiber refers to a fiber formed of one filament, unlike a multifilament fiber. The monofilament fiber may have an average diameter of about 800 denier to about 1,000 denier, but it may have a different average diameter depending on the application. For example, it may have an average diameter of about 200 denier for wig yarn applications; an average diameter of about 800 denier to about 1,000 denier for cable sleeve applications; and an average diameter of about 2,500 denier to about 4,000 denier for paper machine clothing (PMC) applications.

The drying in step (5a) may be carried out in an inert gas atmosphere such as nitrogen. For example, it may comprise pre-crystallization carried out at a temperature of 100°C to 150°C for 2 hours to 5 hours and main drying carried out at a temperature of 140°C or higher for 5 hours or longer. The drying may be carried out until the moisture content in the polyester resin is 200 ppm or less, specifically, 50 ppm or less.

The melt-spinning in step (5b) may be carried out using an extruder equipped with a nozzle, and the melt-spinning temperature condition may be, for example, 275°C to 300°C. The undrawn yarn obtained as a result may have a diameter of, for example, 1,000 denier or less or 500 denier or less, and 30 denier or more.

The drawing in step (5c) may be carried out using a heated drum drawing apparatus or the like and may be carried out, for example, at a drawing ratio of 1.05 to 5.0 at a temperature of 70°C to 180°C. The drawn yarn obtained as a result may have a diameter of, for example, 200 denier or less or 150 denier or less, and 30 denier or more.

The thermal treatment in step (5d) may be carried out, for example, at 100°C to 180°C for 1 minute to 60 minutes.

The polyester fiber according to the present invention may have a tenacity of 3.0 g/denier or more, 3.5 g/denier or more, 4.0 g/denier or more, 4.5 g or more, 5.0 g/denier or more, 5.3 g/denier or more, or 5.4 g/denier or more, and may be 9.0 g/denier or less, 8.0 g/denier or less, 7.0 g/denier or less, or 6.0 g/denier or less, when measured at a speed of 5 mm/minute at 25°C according to ASTM D638.

As a specific example, the polyester fiber may have a tenacity of 3.5 g/denier or more, more specifically, 4.8 g/denier to 9.0 g/denier, when measured at a speed of 5 mm/minute at 25°C according to ASTM D638.

In addition, the polyester fiber may have an elongation at break of 15% or more, 20% or more, 25% or more, 30% or more, 32% or more, or 34% or more, and may be 70% or less, 60% or less, 50% or less, 45% or less, or 40% or less, when measured at a speed of 5 mm/minute at 25°C according to ASTM D638.

As a specific example, the polyester fiber may have an elongation at break of 20% or more, more specifically, 30% to 50%, when measured at a speed of 5 mm/minute at 25°C according to ASTM D638.

In addition, the polyester fiber may have a diameter of 200 denier or less or 150 denier or less, and 30 denier or more.

### Mode for the Invention

Hereinafter, the present invention will be described in more detail with reference to embodiments. However, these examples are provided only for illustration purposes, and the present invention is not limited thereto.

### Example 1

### Step 1: Preparation of recycled bis(2-hydroxyethyl) terephthalate

A first reactor made of stainless steel (SUS) was charged with 2,000 g of a waste polyester resin pulverized to a particle size of 4 mm or less, 4,000 g of ethylene glycol, and 7.0 g of zinc acetate anhydride. The temperature inside the reactor was raised to 180°C, and depolymerization (first glycolysis reaction) was carried out for 2 hours. The reactant (first reactant) thus obtained was transferred to a second reactor and cooled to 150°C. 2,000 g of ethylene glycol was further added thereto, and depolymerization (second glycolysis reaction) was carried out for 2 hours while the reactor temperature was maintained at 150°C.

The reactant (second reactant) thus obtained was cooled to 120°C through reduced pressure flash, and 16 g of a filter aid was added thereto, followed by pressurized filtration to carry out solid-liquid separation. The separated liquid reactant was passed through a column filled with an ion-exchange resin (BC107(H) of Bonlite) to remove ionic impurities to obtain a mixture (third reactant) containing bis(2-hydroxyethyl) terephthalate and ethylene glycol.

The mixture (third reactant) was transferred to a 10-liter distillation apparatus, and vacuum distillation was carried out at 130°C to recover unreacted ethylene glycol. The reactant (fourth reactant) from which ethylene glycol had been removed was subjected to thin film evaporation at 220°C and 0.08 Torr in a thin film evaporator (VKL70-4S of VTA) to obtain 1,040 g of a product from which dimers or higher oligomers had been removed.

Thereafter, for adsorption-crystallization, 1,040 g of the above product and 3,120 g of distilled water were charged to a 20-liter glass reactor, dissolved at a temperature of 70°C, and then 5.2 g of activated carbon was added thereto, followed by stirring for 30 minutes and filtration thereof. The filtrate was cooled to room temperature for the crystallization thereof, filtered, and dried in a vacuum oven. As a result, 1,980 g of a final product containing recycled bis(2-hydroxyethyl) terephthalate was obtained.

### Step 2: Preparation of a solution of recycled bis(2-hydroxyethyl) terephthalate

1,980 g of recycled bis(2-hydroxyethyl) terephthalate (r-BHET), 312 g of water, and 483 g of ethylene glycol (EG) were homogeneously mixed at 70°C to prepare an r-BHET solution (concentration: 71.4% by weight).

### Step 3: Polycondensation reaction under a low vacuum

A 7-liter reactor capable of a reaction under vacuum was charged with 2,775 g of the solution of recycled bis(2-hydroxyethyl) terephthalate (r-BHET) prepared above, 0.8 g of antimony trioxide as a catalyst, 0.6 g of triethyl phosphate as a stabilizer, and 0.4 g of cobalt acetate as a coloring agent. The temperature of the reactor was raised to 190°C over 2 hours. When the temperature reached 190°C, the pressure of the reactor was reduced from normal pressure to 200 Torr (absolute pressure: 200 mmHg) over 30 minutes. A polycondensation reaction under a low vacuum was carried out for 1 hour while the pressure of the reactor was maintained at 200 Torr (absolute pressure: 200 mmHg).

### Step 4: Polycondensation reaction under a high vacuum

The pressure of the reactor was reduced from 200 Torr (absolute pressure: 200 mmHg) to 5 Torr (absolute pressure: 5 mmHg) over 30 minutes. At the same time, the temperature of the reactor was raised to 280°C over 1 hour, and a polycondensation reaction under a high vacuum was carried out while the pressure of the reactor was maintained at 1 Torr (absolute pressure: 1 mmHg) or less. At the beginning of the polycondensation reaction under a high vacuum, the stirring speed may be set high. As the polycondensation reaction under a high vacuum proceeds, when the stirring power is weakened due to the increase in the viscosity of the reactants or the temperature of the reactants rises above the set temperature, the stirring speed may be appropriately adjusted accordingly. The polycondensation reaction under a high vacuum was carried out until the intrinsic viscosity (IV) of the mixture (melt) in the reactor reached 0.64 dl/g. When the intrinsic viscosity of the mixture in the reactor reached the desired level, the mixture was then discharged to the outside of the reactor to form strands, which were solidified with a cooling liquid and then granulated to have an average weight of about 12 to 14 mg.

The granules were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours, and then raised to 220°C at a rate of 40°C/hour and maintained at 220°C. The solid-state polymerization reaction was carried out until the intrinsic viscosity (IV) of the granules in the reactor reached 0.78-0.82 dl/g to prepare a polyester resin.

### Example 2

A polyester resin was prepared by the same procedure as in Example 1, except that, in step 2, 1,980 g of recycled bis(2-hydroxyethyl) terephthalate (r-BHET) and 312 g of water were homogeneously mixed at 70°C to prepare an r-BHET solution (concentration: 86.4% by weight).

### Example 3

A polyester resin was prepared by the same procedure as in Example 1, except that, in step 1, the first glycolysis reaction was carried out at 180°C for 1 hour.

### Example 4

A polyester resin was prepared by the same procedure as in Example 1, except that, in step 1, 2,000 g of a waste fiber was used as a waste polyester raw material.

### Example 5

A polyester resin was prepared by the same procedure as in Example 1, except that, in step 1, 2,000 g of a waste banner was used as a waste polyester raw material.

### Example 6

A polyester resin was prepared by the same procedure as in Example 1, except that, in step 1, no adsorption-crystallization was carried out after the thin-film evaporation.

### Comparative Example 1

A reactor made of stainless steel (SUS) was charged with 2,000 g of a waste polyester resin, 8,000 g of ethylene glycol, and 7.0 g of zinc acetate anhydride. The temperature inside the reactor was raised to 196°C, and depolymerization (glycolysis reaction) was carried out for 4 hours. The reactant thus obtained was cooled to 30°C, and crystallization of bis(2-hydroxyethyl) terephthalate was carried out for 2 hours. The slurry of bis(2-hydroxyethyl) terephthalate and ethylene glycol thus obtained was subjected to solid-liquid separation in a centrifugal separator. Bis(2-hydroxyethyl) terephthalate obtained through centrifugation was washed twice with sufficient distilled water, and the residual solvent was removed in an oven to obtain about 2,020 g of a final product containing bis(2-hydroxyethyl) terephthalate.

The bis(2-hydroxyethyl) terephthalate thus obtained was used to prepare a polyester resin in the same manner as in Example 1.

### Comparative Example 2

A polyester resin was prepared by the same procedure as in Comparative Example 1, except that, in step 1, the depolymerization (glycolysis reaction) was carried out at 210°C.

### Comparative Example 3

A polyester resin was prepared by the same procedure as in Comparative Example 1, except that, in step 1, the depolymerization (glycolysis reaction) was carried out at 196°C, and no adsorption-crystallization was carried out.

### Test Example 1: Evaluation of recycled bis(2-hydroxyethyl) terephthalate

### 1A. Analysis of r-BHET components - HPLC

The components of recycled bis(2-hydroxyethyl) terephthalate (BHET) were analyzed by high-performance liquid chromatography (HPLC).
- About 0.01 g of a sample was diluted in about 20 ml of methanol and then measured by HPLC.
- Model: Waters e2695
- Column: C18 (4.6 × 250 mm), 5 µm
- UV detector: 242 nm
- Injection volume: 10 µl
- Eluent (gradient) A: H₂O + H₃PO₄, B: acetonitrile
- Measured components: bis(2-hydroxyethyl) terephthalate (BHET), mono(2-hydroxyethyl) terephthalic acid (MHET), 2-hydroxyethyl[2-(2-hydroxyethoxy)ethyl] terephthalate (DEG ester 1), bis[2-(2-hydroxyethoxy)ethyl]benzene-1,4-dicarboxylate (DEG ester 2), dimer, trimer, others

### 2B. Residual solvents - GC

The content of residual ethylene glycol (EG) in recycled bis(2-hydroxyethyl) terephthalate (BHET) was measured by gas chromatography (GC).
- About 0.1 g of a sample was diluted in about 10 ml of CHCl₃, treated with a filter of 0.45 µm, and then measured by GC.
- Model: Agilent 7890B
- Column: DB-624 (30 m × 0.25 mm × 1.4 µm)
- Oven Temp.: 60°C (2 min.) - 10°C/min. - 200°C (0 min.) - 20°C/min. - 260°C (5 min.)
- Injector temp.: 250°C
- Detector temp.: 250°C
- Flow: 1.5 ml/min. (N₂), split ratio: 1/50

### 1C. Melting temperature, glass transition temperature, and crystallization temperature - DSC

Differential scanning calorimetry (DSC, Q20 model, TA instrument) was used. Each sample was filled in an aluminum pan, heated up to 280°C at 10°C/minute, maintained at 280°C for 5 minutes, and then cooled down to 30°C at -300°C/minute. Subsequently, the glass transition temperature (Tg) and melting temperature (Tm) were obtained from the heat flow obtained when the temperature was raised to 280°C at 10°C/minute. Subsequently, it was maintained at 280°C for 5 minutes, and the temperature at the apex of the exothermic curve when the temperature was decreased to 30°C at -10°C/minute was taken as the cooling crystallization temperature (Tmc).

### 1D. Inorganic substances - ICP-AES

About 0.3 g of each sample was pretreated with ultrasonic waves and diluted with ultrapure water. Inorganic components were analyzed using inductively coupled plasma atomic emission spectroscopy (ICP-AES, 5100, Agilent) (detection limit: 5 ppm).

### Test Example 2: Evaluation of polyester resins

### 2A. Melting point - DSC

The melting point of each polyester resin was measured using DSC in the same manner as in 1C above.

### 2B. Intrinsic viscosity (IV)

Each polyester resin was dissolved at a concentration of 1.2 g/dl in orthochlorophenol (OCP) at 150°C to obtain a solution, and an Ubbelohde viscometer was used to measure intrinsic viscosity. Specifically, the temperature of the viscous tube was maintained at 35°C, and the time (efflux time) required for the solvent to pass between specific internal sections of the viscous tube and the time required for the solution to pass to obtain specific viscosity, which was used to calculate intrinsic viscosity.

### 2C. Content of DEG - GC

Each polyester resin was pulverized with a grinder, 2 g of the resin was subjected to aminolysis with hydrazine hydrate, and then the content of diethylene glycol (DEG) (% by weight) was measured by gas chromatography (GC).

### Test Example 3: Evaluation of polyester fibers

### 3A. Preparation of a polyester fiber

Each of the polyester resins prepared in the Examples and Comparative Examples was dried in a nitrogen atmosphere for a long period of time to adjust the moisture content to 50 ppm or less. The dried polyester resin was melt-spun through an extruder equipped with a 36-hole nozzle at the tip of the extruder to prepare undrawn yarn of 300 denier. The undrawn yarn was drawn in a heated drum drawing apparatus to obtain a drawn yarn of 60 to 85 denier, which was then thermally treated at 180°CC.

### 3B. Tenacity and elongation at break

For the polyester fibers, tenacity and elongation at break were measured at a measurement speed of 5 mm/minute at 25°C using Zwick Z010 according to ASTM D638.

### 3C. Evaluation of processability

The number of yarn breaks was measured for 5 hours during the spinning process to evaluate the processability according to the following criteria.

| | | | |
|---|---|---|---|
| - ⊚: | 0 yarn break | - ○: | 1 yarn break |
| - Δ: | 2-3 yarn breaks | - ×: | 4 or more yarn breaks |

The results of the Test Examples are shown in the tables below.

**[Table 1]**

| | | | Ex. 1 | Ex. 2 | Ex. 3 | Ex. 4 | Ex. 5 | Ex. 6 |
|---|---|---|---|---|---|---|---|---|
| rBHET | HPLC (area %) | BHET | 98.21 | 98.21 | 98.20 | 97.84 | 96.58 | 96.76 |
| | | MHET | 1.09 | 1.09 | 1.08 | 1.10 | 1.72 | 1.89 |
| | | DEG-ester 1 | 0.44 | 0.44 | 0.43 | 0.62 | 0.61 | 0.80 |
| | | DEG-ester 2 | 0.00 | 0.00 | 0.00 | 0.16 | 0.09 | 0.10 |
| | | Dimer | 0.20 | 0.20 | 0.19 | 0.22 | 0.84 | 0.23 |
| | | Trimer | 0.00 | 0.00 | 0.00 | 0.00 | 0.00 | 0.00 |
| | | Others | 0.06 | 0.06 | 0.10 | 0.06 | 0.16 | 0.22 |
| | GC (% by weight) | Residual EG | 0.07 | 0.07 | 0.07 | 0.08 | 0.07 | 0.23 |
| | DSC (°C) | Tm | 112.2 | 112.2 | 111.9 | 112.1 | 111.5 | 112.5 |
| | ICP-AES (ppm) | Zn | N.D. | N.D. | N.D. | N.D. | N.D. | N.D. |
| | | Sb | N.D. | N.D. | N.D. | N.D. | N.D. | N.D. |
| | | Fe | N.D. | N.D. | N.D. | N.D. | N.D. | N.D. |
| | | P | N.D. | N.D. | N.D. | N.D. | N.D. | N.D. |
| | | Mn | N.D. | N.D. | N.D. | N.D. | N.D. | N.D. |
| | | Mg | N.D. | N.D. | N.D. | N.D. | N.D. | N.D. |
| | | Al | N.D. | N.D. | N.D. | N.D. | N.D. | N.D. |
| | | Co | N.D. | N.D. | N.D. | N.D. | N.D. | N.D. |
| | | Na | N.D. | N.D. | N.D. | N.D. | N.D. | N.D. |
| | | K | N.D. | N.D. | N.D. | N.D. | N.D. | N.D. |
| Resin | DSC | Tm (°C) | 257.8 | 257.2 | 257.4 | 256.7 | 256.7 | 255.2 |
| | IV (dl/g) | | 0.82 | 0.80 | 0.78 | 0.79 | 0.80 | 0.80 |
| | DEG (% by weight) | | 0.6 | 0.7 | 0.7 | 0.8 | 0.8 | 0.8 |
| Fiber | Tenacity (g/denier) | | 5.5 | 5.2 | 5.2 | 5.3 | 5.1 | 5.0 |
| | Elongation at break (%) | | 35.7 | 34.7 | 36.6 | 33.1 | 35.1 | 35.0 |
| | Processability | | ⊚ | ⊚ | ⊚ | ○ | ○ | ○ |

**[Table 2]**

| | | | C. Ex. 1 | C. Ex. 2 | C. Ex. 3 |
|---|---|---|---|---|---|
| rBHET | HPLC (%) | BHET | 82.55 | 84.58 | 90.68 |
| | | MHET | 1.00 | 2.18 | 1.66 |
| | | DEG-ester 1 | 3.67 | 5.35 | 2.88 |
| | | DEG-ester 2 | 0.38 | 0.27 | 0.25 |
| | | Dimer | 10.44 | 6.36 | 3.68 |
| | | Trimer | 0.89 | 0.72 | 0.35 |
| | | Others | 1.07 | 0.54 | 0.50 |
| | GC (% by weight) | Residual EG | 0.41 | 0.45 | 0.33 |
| | DSC (°C) | Tm | 110.5 | 108.5 | 111.1 |
| | ICP-AES (ppm) | Zn | 80 | 50 | N.D. |
| | | Sb | 5 | 8 | N.D. |
| | | Fe | 11 | 7 | N.D. |
| | | P | 22 | 23 | N.D. |
| | | Mn | N.D. | N.D. | N.D. |
| | | Mg | N.D. | N.D. | N.D. |
| | | Al | N.D. | N.D. | N.D. |
| | | Co | N.D. | N.D. | N.D. |
| | | Na | N.D. | N.D. | N.D. |
| | | K | N.D. | N.D. | N.D. |
| Resin | DSC (°C) | Tm | 250.5 | 248.5 | 252.1 |
| | IV (dl/g) | | 0.81 | 0.80 | 0.79 |
| | DEG (% by weight) | | 3.4 | 4.5 | 2.0 |
| Fiber | Tenacity (g/denier) | | 4.2 | 3.8 | 4.7 |
| | Elongation at break (%) | | 38.7 | 33.8 | 35.4 |
| | Processability | | △ | × | △ |

As can be seen from the above tables, the recycled bis(2-hydroxyethyl) terephthalate (rBHET) prepared in step 1 of Examples 1 to 6 had high purity and a low content of impurities such as diethylene glycol esters (DEG esters) and dimers, and no inorganic impurities were observed. As a result, the polyester resins prepared in Examples 1 to 6 each had a low content of diethylene glycol (DEG), high melting point, and excellent structural regularity; thus, they were excellent in both tenacity and processability when spun into a fiber.

In particular, in Example 1 in which ethylene glycol was further added in the preparation of the solution of recycled bis(2-hydroxyethyl) terephthalate in step (2), it showed better properties than those of Example 2, which was not the case.

In contrast, the recycled bis(2-hydroxyethyl) terephthalate (rBHET) prepared in step 1 of Comparative Examples 1 and 3 contained a large amount of dimers or diethylene glycol esters (DEG esters); thus, the polyester resins prepared therefrom had a high content of diethylene glycol (DEG), a low melting point, and insufficient structural regularity, resulting in poor tenacity and poor processability during fiber spinning.

## Claims

1. A process for preparing a polyester resin, which comprises:
(1) depolymerizing waste polyester to prepare recycled bis(2-hydroxyethyl) terephthalate, wherein the recycled bis(2-hydroxyethyl) terephthalate has a peak area fraction of bis(2-hydroxyethyl) terephthalate of 96% or more and a peak area fraction of a diethylene glycol ester of less than 2% in total when measured by high-performance liquid chromatography (HPLC);
(2) mixing the recycled bis(2-hydroxyethyl) terephthalate with a solvent to prepare a solution of recycled bis(2-hydroxyethyl) terephthalate;
(3) subjecting the recycled bis(2-hydroxyethyl) terephthalate solution to a first polycondensation reaction under a pressure of 200 mmHg to 600 mmHg to prepare an oligomer; and
(4) subjecting the oligomer to a second polycondensation reaction under a pressure of less than 200 mmHg to prepare a polyester resin.

2. The process for preparing a polyester resin of claim 1, wherein the recycled bis(2-hydroxyethyl) terephthalate in step (1) is prepared by a process, which comprises:
(1a) subjecting the waste polyester to depolymerization by a first glycolysis reaction at a temperature of 180°C to 200°C to obtain a first reactant;
(1b) subjecting the first reactant to depolymerization by a second glycolysis reaction at a temperature of 150°C to 170°C to obtain a second reactant;
(1c) subjecting the second reactant to ion exchange through an ion-exchange resin to obtain a third reactant;
(1d) removing an unreacted glycol from the third reactant through distillation at a temperature of 150°C or lower to obtain a fourth reactant; and
(1e) subjecting the fourth reactant to distillation to obtain crude bis(2-hydroxyethyl) terephthalate.

3. The process for preparing a polyester resin of claim 1, wherein the recycled bis(2-hydroxyethyl) terephthalate in step (1) has a peak area fraction of oligomers of 3% or less in total when measured by high-performance liquid chromatography.

4. The process for preparing a polyester resin of claim 1, wherein the concentration of the solution of recycled BHET in step (2) is 50% by weight to 95% by weight.

5. The process for preparing a polyester resin of claim 1, wherein the solvent comprises at least one of water and ethylene glycol.

6. The process for preparing a polyester resin of claim 1, wherein the first polycondensation reaction in step (3) is carried out at a temperature of 180°C to 250°C and a pressure of 200 mmHg to 400 mmHg.

7. The process for preparing a polyester resin of claim 1, wherein the second polycondensation reaction in step (4) is carried out at a temperature of 250°C to 300°C and a pressure of 0.01 mmHg to 150 mmHg.

8. The process for preparing a polyester resin of claim 1, wherein the process for preparing a polyester resin further comprises subjecting the polyester resin to a solid-state polymerization after step (4).

9. The process for preparing a polyester resin of claim 8, wherein the solid-state polymerization is carried out at a temperature of 200°C to 220°C.

10. A polyester resin, which is prepared by the process of claim 1 and has an intrinsic viscosity of 0.6 dl/g to 1.2 dl/g.

11. The polyester resin of claim 10, wherein the polyester resin has a concentration of diethylene glycol of 0.8% by weight or less when measured by gas chromatography.

12. A polyester fiber, which comprises the polyester resin of claim 10.

13. The polyester fiber of claim 12, wherein the polyester fiber has a tenacity of 3.0 g/denier or more when measured at a speed of 5 mm/minute at 25°C according to ASTM D638.

14. The polyester fiber of claim 12, wherein the polyester fiber has an elongation at break of 20% or more when measured at a speed of 5 mm/minute at 25°C according to ASTM D638.

15. A process for preparing a polyester fiber, which comprises:
(1) depolymerizing waste polyester to prepare recycled bis(2-hydroxyethyl) terephthalate, wherein the recycled bis(2-hydroxyethyl) terephthalate has a peak area fraction of bis(2-hydroxyethyl) terephthalate of 96% or more and a peak area fraction of a diethylene glycol ester of less than 2% in total when measured by high-performance liquid chromatography (HPLC);
(2) mixing the recycled bis(2-hydroxyethyl) terephthalate with a solvent to prepare a solution of recycled bis(2-hydroxyethyl) terephthalate;
(3) subjecting the recycled bis(2-hydroxyethyl) terephthalate solution to a first polycondensation under a pressure of 200 mmHg to 600 mmHg to prepare an oligomer;
(4) subjecting the oligomer to a second polycondensation under a pressure of less than 200 mmHg to prepare a polyester resin; and
(5) spinning the polyester resin to obtain a fiber.
